# EUROPEAN PATENT APPLICATION

(11) **EP 1 760 144 A2**
(43) Date of publication of application: **07.03.2007**
(21) Application number: 05750573.7
(22) Date of filing: 20.05.2005
(51) Int. Cl.: C12N 5/06

(54) **CARTILAGE AND BONE REPAIR COMPOSITION**

(30) Priority: 21.05.2004 ES 200401227
(71) Applicant: Universidad de Málaga, 29071 Málaga (ES)
(72) Inventor: BECERRA RATIA, José Dpto.Biología Celular, E-29071 Malaga (ES); ANDRADES GOMEZ, José, A., Dpto.Biología Celular, E-29071 Malaga (ES); CIFUENTES RUEDA, Manuel Dpto.Biología Celular, E-29071 Malaga (ES); GUERADO PARRA, Enrique Servicio de Cirugía Ortop., E-29600 Marbella (ES)
(74) Representative: Carpintero Lopez, Francisco
(86) International application number: PCT/ES2005/000287
(87) International publication number: WO 2005/112542

(57) **Abstract**

The invention relates to a cartilage and bone repair composition comprising a group of human mesenchymal stem cells that are differentiated to the chondro-osteogenic lineage, by means of the amplification thereof with human serum and a transforming growth factor-beta 1 with a molecular domain for binding to collagen I (TGF-ß1-DUC) obtained in eukaryotic expression systems, and a biocompatible material which is absorbed by the cells thus treated. The inventive composition can be employed using implants in the area to be repaired or it can be employed directly by injecting the cells in suspension either at the site of the injury or into the systemic circulation for the widespread distribution thereof

## Description

### FIELD OF THE TECHNIQUE

The present invention is applicable in the biomedical industry dealing with the manufacture of agents involved in skeletal repair and more specifically in cartilage and bone repair processes of any etiology, spinal fusion or arthrodesis, osteosynthesis, prosthesis for arthroplasties, osteoporosis, etc. and any other requiring repair or regeneration of bone or cartilage tissues and where a supplemental supply of cells in differentiation involves effective help for achieving the tissue repair involved.

### PREVIOUS STATE OF THE ART

Tissue regeneration is a complex process that involves the culmination of a large variety of cells that were present or have been recruited at the site of the lesion. All events occurring in the repair process are guided by a number of interactions between growth factors and cytokines, cells and molecules forming the extracellular matrix.

A variety of inducer signals and growth factors including TGF-β, PDGF, BMPs, IGFs and FGFs coexist in the bone (Becerra et al. Med Clin 2001;116:23-24). Among them, only BMPs are osteoinducers; however all are involved in the repair of a given fracture (Reddi Nature Medicine 1998, 3:837-839; Groeneveld and Burger Eur J Endocrinol 2000; 142:9-21). Also in the bone there are osteogenic precursor cells that respond to the stimulus of the fracture, activating and producing BMPs that induce the migration of mesenchymal stem cells (MSCs), which proliferate and are differentiated to bone-forming cells (Guerado et al. Rev Ortop Traumatol. 2003; 47, 362-374). However, the understanding of the complex interrelation between mesenchymal stem cells and osteoprogenitor precursor cells with the biochemical signals modulating their migration, proliferation and differentiation to the osteogenic lineage, still shows significant gaps.

TGF-βs are a group of growth factors coded for a gene family expressed in multiple tissues and species (Kingsley Genes Dev; 1994;8:133-146; Bostrom and Asnis, Clin Orthop Relat R, 1998; 355S:124-131) Bone was one of the first tissues where local production of a TGF-β with the ability to regulate cell function was seen (Centrella et al. J Bone Joint Surg 1991; 73-A: 1418-1428). Although platelets are the main source of TGF-β, bone is the most significant reservoir, 100 times greater than in other soft tissues, where there is also a shared presence of BMPs (Roberts et al. Biochemistry 1983;22:5692-5698).

Significant results have been obtained in recent years, sometimes contradicting, when adding TGF-β to osteoblastic cells in culture. Their mythogenic or osteodifferentiating effect, or the contrary, has been attributed to cell heterogeneity in cultures, presence of other growth actors, cell density, culture conditions or a possible biphasic effect of the factor (Noda et al., Endocrinology 1988;124: 612-617; Stein and Lian, Cellular and Molecular Biology of Bone. San Diego: Academic Press; 47-95 1993). Recent studies also consider the possible existence of different target cells for TGF-β or BMPs; while the former appear to act on stem cells already involved in the osteogenic lineage, BMPs can act over non-compromised cells (Ballock et al., J. Oprthop. Res. 1997;15:463-467). In any case, it appears to be clear that MSCs can perform self-renewal and amplification in the presence of TGF-β1 (Andrades et al., Cells. Exp Cell Res, 1999;250:485-498).

To control bioavailability and to assure the presence with some control of TGF-β1 in MSC cultures, an experimental procedure has been recently developed whereby, from rat bone marrow, a cell population has been isolated, amplified and induced to osteogenic lineage when cultured in collagen gels, in the presence of a TGF-β1 with a molecular domain for specific binding to collagen I. It has been proposed that the domain for binding to collagen allows for a slow release of the active factor in the gel to which it is bound, prolonging its half-life and enhancing its availability for acting on target cells (Andrades et al. Cells. Exp Cell Res, 1999;250:485-498).

It has been demonstrated that bone regeneration capacity decreases with age. While the number of hematopoietic cells of the bone marrow persists over life, its MSCs decrease substantially, in addition to noticing some difficulty of progression for the lack of osteoinducing factors or inability of the cells to produce or respond to them (Haynesworth et al., Musculoskeletal Soft-Tissue Aging: Impact on Mobility. Section 1, Chapter 7. Rosemont: AAOS, 80-86, 1994). Therefore, it is desirable to develop procedures that enable the selection of MSCs of individuals with bone repair inability, their amplification in vitro and capacitation to osteogenic lineage.

After performing the above reliably, the cells thus treated should be transferred to the sites where the patient requires skeletal repair, vehicling them with the appropriate biomaterial, and thus developing a kind of autologous cell therapy which enables to establish effectively the osteo-repair function lost or seriously jeopardized. This also avoids the undesirable effects of systemic or local infection of bioactive factors, as controlling them beyond the site of repair for the moment poses difficulties and their pleiotropic effects are a threaten not to be missed.

In any case, the transplant of capacitated cells should be made in a microenvironment that promotes osteogenesis, when the consolidation of two or more bone fragments is required. With this regard, when the fracture or non-consolidation focus (pseudo-arthrosis) is characterized by being hypervascular with proliferation of chondro-osteogenic tissue (no binding or hypertrophic pseudo-arthrosis), the simple rigid non-dynamic stabilization of the focus by intra or extramedullary implants should promote consolidation. In these cases, the transplant of capacitated cells to the osteogenic lineage over a carrier of hydroxyapatite or collagen finds a stable hypervascular bed after osteosynthesis, an ideal microenvironment for the development of the osteogenic callus. In the cases of non-consolidation or atrophic pseudo-arthrosis, the fibrous tissue between the two fragments should be removed, leaving bone with bone and searching for a vascular source by bone perforations and lining with vascularized tissue (generally muscle). Both fragments must be stabilized by rigid osteosynthesis that prevents mobility in the bone interface, as in case of movement in the interface, it will be destroyed and will prevent vascular proliferation, and will stimulate the expression of chemical mediators inducing chondrogenesis instead of osteogenesis (Rüedi and Murphy, AO Principles of fracture management. Sttugart, Georg Thieme Verlag. 2000; Browne et al., Skeletal Trauma, Basic Science, Management, and Reconstruction. Philadelphia, Saunders, 2003).

It is also desirable to develop procedures to increase the ability of bone mass formation in individuals with osteoporosis, by procedures that involve an intervention in the imbalance in these patients between bone formation and resorption, to the detriment of the former. Systemic injection of capacitated cells in vitro to the osteogenic lineage will allow for balancing this equation.

### EXPLANATION OF THE INVENTION

The present invention has a clear application in the preparation of agents and compositions to be used for skeletal repair and more specifically in cartilage, bone repair processes, osteoporosis, prostheses for arthroplasties, etc. that can be injected *in situ* in small non-joining fractures or in cartilaginous lesions, in the systemic circulation of osteoporotic individuals or can be implanted adsorbed in the appropriate biomaterial (collagen, hydroxyapatite, etc.) to promote cartilage or bone formation in lesions of some significance. Furthermore, they can be adsorbed in the hydroxyapatite (periapatite) coating the prostheses for hip, knee arthroplasties to enhance biological integration of these prostheses in the host, prolonging their life. These preparations can be also used in spinal arthrodesis for promoting spinal fusion and improve the efficiency of these procedures performed by the standard procedures with autologous graft or bone bank.

In a first aspect of this invention, this provides a method to increase bone and/or cartilage formation in humans in need of it by administering human mesenchymal stem cells differentiated to the chondro-osteogenic lineage by means of amplification with human serum and a transforming growth factor-beta 1 with a molecular domain for binding to collagen I (TGF-β1-CBD) obtained in eukaryotic expression systems.

In a preferred embodiment of this invention, the administration of these capacitated cells is performed by injection in situ, into the systemic circulation or by implants, adsorbed in the appropriate biomaterial. The preferred embodiment is using implants with the cells adsorbed in the appropriate material, and an even more preferred embodiment is the use of reabsorbable hydroxyapatite, with 50% porosity, diffusion chambers of collagen gels.

In a preferred embodiment of this invention, human MSCs have been helped in their induction to the chondro-osteogenic lineage using a glucocorticoid. The use of dexamethasone is preferred. In a more preferred embodiment this induction is performed in the presence of dexamethasone and β-glycerophosphate.

In another preferred embodiment of this invention, the transforming factor-beta 1 joined with a molecular domain for binding to collagen I has been expressed in eukaryotic expression systems. A preferred embodiment is the expression using insect cells, and even more preferred the use of insect cells transfected with baculovirus.

In a second aspect of this invention, a capacitation procedure in vitro is provided with chondro-osteogenic cells from human MSCs, which comprises the following steps:
- the primary culture of stem cells in a medium supplemented with human serum and a transforming growth factor-beta 1 with a molecular domain for binding to collagen I (TGF-β1-CBD);
- subsequent amplification by supplementation with human serum and TGF-β1-CBD;
- chondro-osteogenic induction with dexamethasone and β-glycerophosphate.

Human MSCs can be obtained from different tissues and locations of the patient and from bone marrow, umbilical cord blood, peripheral blood or internal mass of frozen human blastocytes.

In a preferred embodiment of this capacitation procedure, the transforming growth factor-beta 1 fused with a molecular domain for binding to collagen I has been expressed in eurokaryotic expression systems. A more preferred embodiment is the use in insect cells for expression. And an even more preferred embodiment is the use of insect cells transfected with baculovirus.

According to another preferred embodiment, the primary culture is performed in a human type I collagen gel.

In another preferred embodiment, the selection of bone marrow MSCs is performed in a culture supplemented with a quantity between 0.1 and 1% of human serum, preferably 0.5%.

According to a preferred embodiment, the amplification occurs during a period ranging between 1 and 25 days, during which the culture medium is supplemented with a quantity between 5% and 25% of human serum, preferably 20%.

In a third aspect of this invention, this provides cells with chondro-osteogenic capacity obtainable by the aforementioned capacitation procedure, which comprises the selection, amplification and chondro-osteogenic induction of MSCs.

In a fourth aspect of this invention, the use of cells with chondro-osteogenic capacity is provided, obtainable by the aforementioned capacitation procedure, in the preparation of a composition for bone and/or cartilage repair in humans.

In a fifth aspect of this invention, a composition that can induce osteogenesis is provided that comprises a biocompatible and osteoconductive material, and a group of cells with chondro-osteogenic capacity obtainable by the aforementioned capacitating procedure.

### BRIEF DESCRIPTION OF DRAWINGS

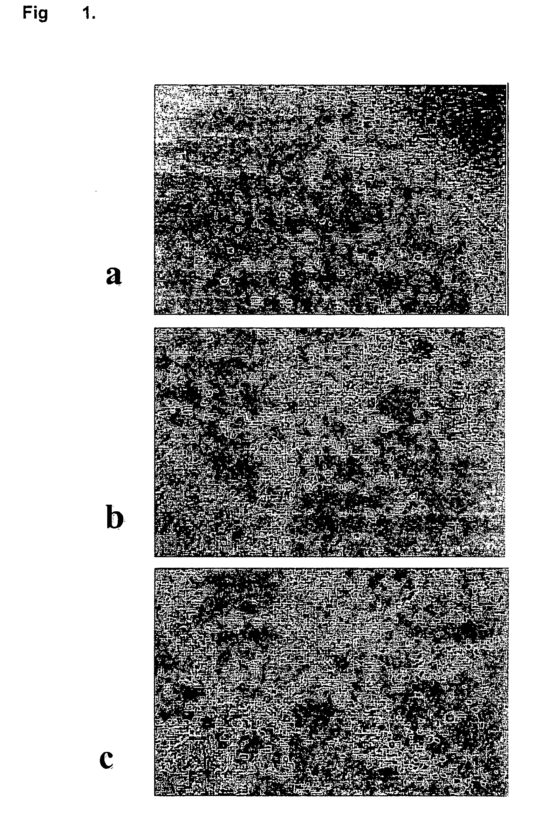

Figure 1.- a) MO cells cultureed in vitro for 10 days in collagen gel in the presence of rhTGF-β1-CBD. Very few isolated cells are seen. MO cells cultured for 35 days in collagen gel (b and c), in the presence of rhTGF-β1-CBD and induced with dexamethasone and β-glycerophosphate, respectively. A greater cell density and some colonies of them are seen. x60.

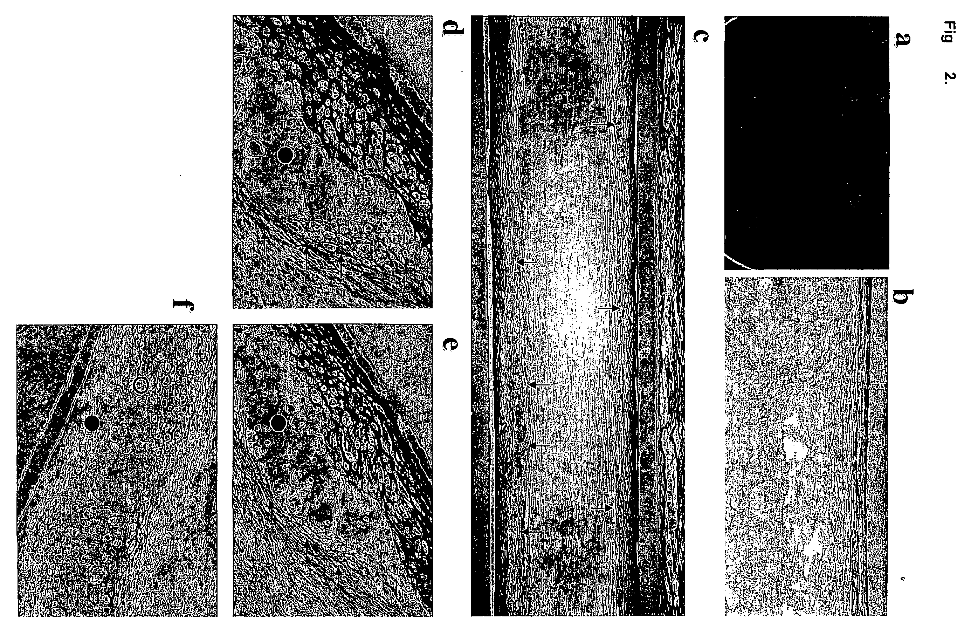

Figure 2.- a) shows radiographs of four diffusion chambers after the period of implantation in rat subcutaneous tissue. The different densities show different quality and amount of neoformed tissue. b) Shows of a histological section of a chamber after the implantation period, where cells cultured under control conditions were placed, without rhTGF-β1-CBD; inside they are full of more or less fibrous materials, finding no chondro-osteoid tissue accumulations (asterisk, chamber filter). Sirius red-hematoxylin. x10. c) Appearance of the section of a chamber at low magnification, where cells processed in vitro were placed in the presence of rhTGF-β1-CBD, detecting several condensations of neoformed tissue (arrows) close to the filters (asterisk) standing up from the others. Sirius red-hematoxylin. x10. d) Detail of one of these condensations differentiating two classes of tissue, one of cartilaginous appearance (filled circle), poorly stained, and another intensely stained, of osteoid appearance (empty circle). Outside of the pale tissue, more stained fibers are seen, which resemble a perichondrium (arrows). Sirius red-haematoxylin. x40 e) A section parallel to the above is presented, stained with Goldner trichromic that shows an intense blue color (empty circle), the most dense tissue (osteoid) and in pale color something that looks like a cartilage (filled circle). Around the circle there are some bluish fibers that appear to be a perichondrium layer (arrows). x40 f) When another parallel section is stained with alcian blue, the cartilaginous tissue appears blue (filled circle), while the osteoid appears colorless (empty circle), as well as the perichondrium. x40. This is consistent with a chondro-osteoid neoformation. Both sirius red and the trichromic stain intensely collagen, particularly of type I, characteristic of bone tissue and the perichondrium, while alcian blue stains the acid and sulfated groups of proteoglycans, abundant in the cartilaginous matrix.

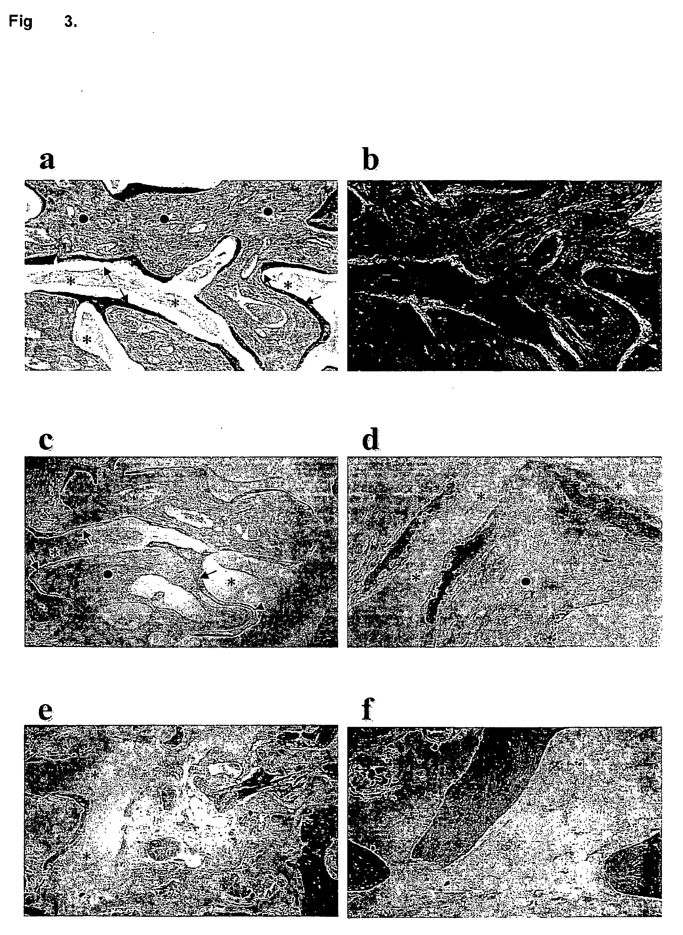

Figure 3.- Sections of tissue formed where the hydroxyapatite implants were performed with cells processed as indicated in the text. As the material was decalcified before cutting and coloring, the empty spaces (asterisks) correspond to the walls of hydroxyapatite trabeculae. The hollows in these walls are now filled with a tissue that can be called medullary (filled circles), while attached to these trabeculae there are thin neoformed tissue sheets (arrows). a) shows how the tissue lining the hydroxyapatite walls is rich in collagen (intense red with sirius red) (arrows), as shown by the birefringence in b) when lighting the same section with polarized light. This collagen is of type I, as shown by its positive nature (arrows), that appears when these tissue sections are treated with anti-collagen I antiserum shown in c) x20. d) shows, with greater magnifications, that the new tissue contains cells inside, like osteoblasts that are trapped in the matrix they synthesize (Goldner trichromic). The new tissue shows a red color (more mature tissue) or greenish color (immature tissue). X40. e) shows a section of an area close to the implanted hydroxyapatite (asterisks) where it can be seen that several trabeculae of host tissue show new appositions of green-bluish young bone, which indicates some osteogenic activity, not only where we placed HA, but also around it, which indicates "some distance osteoinduction" probably caused by the cells "diffusing" HA (Goldner trichromic). X20. On the contrary, f) shows the section of an area of the same host bone, far from the places of the HA fragments, where it is seen that bone trabeculae do not show new tissue incorporation, and, therefore, that osteoinduction did not reach further the area of the implants. x20.

### EXAMPLES

This invention is illustrated by the following non-limiting examples.

### Example 1- Design and obtention of the human fusion recombinant protein TGF-β1 with a collagen I binding domain (rhTGF-β1-CBD).

The cDNA for the TGF-β1 gene was obtained by RT-PCR, using a DNA-polymerase with corrective capacity. The mold used was total RNA, extracted by the method of Chomczynski and Sacchi (1987), from human osteosarcoma cells MG63 (ref. in ATCC: CRL-1427). The primers are designed with the aid of a software (Oligo v6.6) from the mRNA sequences published in the genbank. The PCR products were cloned "in dull" in a maintenance plasmid (pBSK II, Stratagene). Once the genes were cloned, they were checked by sequentiation of both threads. Using these plasmids as mold, they were amplified by PCR with pfu-DNA-polymerase, the region coding for the mature peptide. The primers used contain the Collagen-Binding Domain (CBD) and restriction sites adequate to clone directionally in a transfer plasmid of an expression system in baculovirus, containing a sequence for a peptide signal of secretion, specific for insect cells (pAcGP67B, Pharmingen). To obtain the factor without CBD, the primers had only the restriction sites. The chain sense primer had the following structure: *5'- (BamHl)-sequence of CBD-GGAGGA-(Nhel)-6 first mature peptide nucleotides TGF-β1.* The chain antisense primer was formed by several nucleotides of the 5' end of the gene and comprised the stop codon. In addition it has the Not I site. In all cases, the structure was checked by sequencing the PCR product or the transfer plasmid containing it.

Once the transfer plasmid for the factor was available with and without CBD, insect cells were cotransfected (sf9) with a mixture made up by the transfer plasmid and a commercial DNA baculovirus (BacVector3000, Novagen), that has removed besides the polyhedrin gene, other genes coding for cysteine-proteases. We use a liposome-mediated transfer method (lipofectin, Novagen) according to the instructions of the commercial firm.

Once the DNAs were transferred to the cells, they were cultured in TNM-FH medium for several days, until cell affectation was seen. During this time, an homologous recombination occurs inside the cells whereby the viral DNA is transferred downstream the polyhedrin promoter, a region of the plasmid comprising the peptide sequence signal plus that of the factor with or without CBD. This process generates infecting virus that kill the cell, are released to the environment and infect other cells.

A recombinant virus was selected from the mixture of recombinant virus in the culture supernatant. This was performed by the plate test that consists of sowing a semiconfluent cell culture with dilutions of the transfection supernatant. After the infection period (1 h), the plate was covered with agarose dissolved in culture medium and was left in the culture for 4 days. With this, it was obtained that the diffusion of the infection occurs in the environment of some cells, which generated a lysis plate. These lysis plates were removed individually and placed in a small volume of medium to extract the virus. Based on the no. and size of the lysis plates of the first selection, this procedure was repeated once again from a previous plate of lysis and we will thus be sure to have a cloned recombinant virus. Once this virus was amplified, it was analyzed by PCR with the specific primers for the factor, to check that it contained the relevant gene. Then it was necessary to use a large-volume stock that should be titrated, to establish the no. of pfu/mL (plate forming units/mL).

From this point, for each recombinant virus, the appropriate infection conditions were established in order to obtain an optimum production. For this, 12-well plates were sown with a known number of cells. In them it was planned to sow different percentages of no. of virus/cell (MOI, multiplicity of infection) in addition to different culture times. MOIs of 0.1, 1 or 10 were tested, sampling at 48, 72 and 96 h of incubation. In order for the serum of the culture medium not to be contaminated with undesirable proteins, after the first 24 hours after infection, the culture was continued with serum-free medium or with a very low percentage of serum. After completing each experimental situation, the supernatant was collected and centrifuged to remove cell rests.

The well cells were broken by freezing-unfreezing and extracted with PBS. Both samples were analyzed by SDS-electrophoresis (denaturalizing conditions), with no reducing agents. The polyacrylamide gel was stained with Coomassie blue (if not sufficiently sensitive, staining with silver can be performed). The different samples were also analyzed by immunoblotting using specific TGF-β1 antibodies. We used a secondary antibody labeled with peroxidase and identified its presence by chemoluminescence (CSPD, Amersham). With this information we evaluated the percentage of monomer/dimmer produced and the yield of the system.

To obtain a significant amount of recombinant proteins, a liquid culture of approximately 250 mL was started, with about 500,000 cells/mL. When a growth of about 2x106 cells/mL was reached, it was inoculated with the virus, using the highest MOI and with the greatest yield. At 24 hours, the medium with serum was removed and the cells were cultured with serum-free medium, for a number of days determined by previous tests. After this time, the proteins were purified according to the protocol described by Aono et al. (1995). The method uses cation exchange chromatography, using a SP-Sepharose column (Amersham-Pharmacia). The culture medium was balanced at pH 8 and urea 4M was added. The column was balanced with Tris-HCl 20 mM pH8 buffer, urea 4 M. After running the medium through the column, it was washed with the buffer and the proteins were eluted with a linear gradient of NaCl between 0 and 0.7 M. For the different fractions, absorbance was measured at 280 nm and those with proteins were analyzed by electrophoresis, following the above described technique. The positive fractions were gathered and concentrated by ultrafiltration and used in biological activity assays. The concentration of proteins of this final fraction was measured by Bradford.

### Example 2.- Obtaining and preparing human mesenchymal stem cells.

Human mesenchymal stem cells were obtained from human bone marrow (BM) after surgical extraction of the iliac crest from informed donors. The marrow tissue was washed in complete medium (-MEM, GIBCO, HD, US), with antibiotics (100 mg/mL penicillin G (sigma), 50 mg/mL gentamycin (sigma) and 0.3 mg/ml of fungizone (Flow-ICN)). The suspension of individualized cells was obtained after sequential dispersing through needles of gauge 18, 20 and 22, and finally filtered through 20 micrometer Teflon sterile filters (Cell Strainer, Falcon, Lincoln Park, NJ) for separating tissue rests and cell accumulations.

### Example 3.- Culture of MSCs in collagen gels

The cell suspension obtained according to example 2 was centrifuged at 100 g for 5 min. The centrifuge was resuspended in culture medium with a very low concentration of serum of the patient (HS, 0.5%).

Before placing the cells in the culture wells, plates of 48, 100 mL of a human collagen I solution (Sigma-Aldrich) were placed at the bottom of them, at a concentration of 0.35 mg/mL, at pH 7.4. 150 mL per well of a mixture of collagen I, TGF-β1 at a concentration of 1 mg/mL, and 2x10⁶ bone marrow cells were placed over a layer. The culture plates were placed in the culture oven at 37°C for 15 minutes to allow for formation of the collagen gel that was liquid until that time. Then 10 mL per well of culture medium were placed, also containing the same concentration of TGF-β1. The cultures thus arranged were cultured in an atmosphere of 95% of air and 5% of CO₂, at 37°C and 100% relative humidity. Every 3 days, the culture medium was changed, adding in each case the same concentration of TGF-β1.

The optimum concentration of TGF-β1 was investigated in previous tests on chemotactic response. For each experimental condition, 150 mL of collagen (0.35 mg/mL) were mixed, with the concentration of the factor previously selected as having the greater chemotactic capacity. Confluent rat bone marrow cultures were dissociated and the cells were placed in the upper compartment of the Boyden chambers (Neuroprobe, Rockville, MD) at a concentration of 2x10⁵ cells/mL of -MEM. The lower compartment of the chamber contained the medium with the different concentrations of the test factor. After 4 h of incubation at 37°C in 5% CO₂, the filters were removed and fixed in methanol; they were washed, stained and mounted in slides. Chemotaxis was quantitated counting the number of cells migrating in 20 fields of the microscope (x400) in each filter.

### Example 4.- Experimental procedure for capacitation of cells (selection, amplification and induction).

MO cells were maintained under the experimental conditions described above for 10 days, changing the culture medium, and, therefore, the TGF-β1, every 3 days. After these 10 days, the culture medium was implemented with 10% of serum of the patient (HS 10%). This completes the so-called selection period and starts the amplification period, that was maintained for the time necessary to obtain an amount of cells that enable the transplant, but in any case, this period should not exceed the 25 days. After this period, the medium was implemented with dexamethasone (10⁻⁸ M) and β-glycerophosphate (2 mM) that act as osteoinducing chemical agents. This period of only 2 days is called induction.

During the time of cell culture, samples were taken at days 0, 10, 14, 18, 21, 24, 27, 30, 33 and 36, and analyses were performed on the no. of cells and presence of alkaline phosphatase and calcium content. Tables I, II, III show the values obtained. They show how TGF-β1-CBD increases the number of cells vs the control. The same occurs with the synthesis of alkaline phosphatase. Calcium content also increases with the culture days, while the values are unnoticeable in the control.

The microscopic examination of the cultures during the process described shows how cells proliferate during the culture days and even form colonies during the amplification period, when they are in the presence of TGF-β-1DUC (Figure 1). When the cells are treated with the commercial TGF-β1 (R&D Systems, Minneapolis, MN), they are disperse or poorly clustered.

**TABLE 1. Effect of the culture conditions on cell replication (DNA µg)**

| **Culture days** | | | | | | | | | | |
|---|---|---|---|---|---|---|---|---|---|---|
| | 0 | 10 | 14 | 18 | 21 | 24 | 27 | 30 | 33 | 36 |
| Control | 28.5 ± 1.55 | 2.9 ± 0.67 | 4.9 ± 0.52 | 6.6 ± 0.91 | 8.2 ± 0.86 | 8.5 ± 0.86 | 8.5 ± 0.74 | 9.0 ± 0.70 | 9.7 ± 0.88 | 10.6 ± 0.76 |
| rhTGF-β1-F2 | 0.0 + 0.00 | 6.8+ 0.89 | 14.1 ± 2.74 | 19.5 ± 2.79 | 20.3 ± 1.49 | 22.8 ± 1.49 | 25.4 ± 2.10 | 27.8 ± 2.23 | 29.1 ± 2.00 | 31.7 ± 2.56 |
| The data correspond to the mean values and their ± SD for every four samples. | | | | | | | | | | |

**TABLE 2. Effect of the culture conditions on the activity of alkaline phosphatase (U AP/µg DNA)**

| **Culture days** | | | | | | | | | | |
|---|---|---|---|---|---|---|---|---|---|---|
| | 0 | 10 | 14 | 18 | 21 | 24 | 27 | 30 | 33 | 36 |
| Control | 2.2 ± 0.20 | 0.2 ± 0.01 | 1.0 ± 0.03 | 1.4 ± 0.06 | 1.4 ± 0.05 | 1.5 ± 0.07 | 1.5 ± 0.09 | 1.5 ± 0.12 | 1.4 ± 0.10 | 1.44 ± 0.11 |
| rhTGF-β-F2 | 2.2+ 0.20 | 1.0+ 0.03 | 2.91 ± 0.27 | 3.8 ± 0.21 | 3.43 ± 0.17 | 3.58 0.15 | 3.5 ± 0.21 | 4.2 ± 0.28 | 3.61 ± 0.24 | 3.18 0.19 |
| The data correspond to the mean values and their ± SD for every four samples. | | | | | | | | | | |

**TABLE 3. Effect of the culture conditions on calcium content (µg Ca/well)**

| **Culture days** | | | | | | | | | | |
|---|---|---|---|---|---|---|---|---|---|---|
| | 0 | 10 | 14 | 18 | 21 | 24 | 27 | 30 | 33 | 36 |
| rhTGF-β1-F2 | ND | ND | ND | 0.045 ± 0.07 | 0.062 ± 0.08 | 0.075 ± 0.06 | 0.086 ± 0.09 | 0.170 ± 0.01 | 0.171 ± 0.01 | 0.173 ± 0.01 |
| ND, not detected, p < 0.01 vs the control cultures. The data correspond to the mean values and their ± SD for every four samples. | | | | | | | | | | |

### Example 5.- Transfer of capacitated cells

After completing the culture period, the collagen gels were targeted with collagenase and the cells were dispersed with 0.05% trypsin and 0.02% EDTA at 27°C. The cells were centrifuged, washed and resuspended in serum-free medium. After the appropriate count, part of them were inserted in Millipore diffusion chambers implanted in rat subcutaneous tissue and the other part were absorbed in hydroxyapatite fragments that are implanted in a specific bone lesion (Figure 3).

### Example 6.- Subcutaneous implantation. Formation of ectopic bone.

The diffusion chambers with 1x10⁶ human cells capacitated by the procedure described and suspended in serum-free medium were implanted subcutaneously in the back of Fisher rats, 344 females aged 7 months, after administering them anesthesia with a subcutaneous injection of pentobarbital sodium 3.5 mg/100 mg body weight. A 2 cm cut was performed in the midline of the back to insert the chambers. Each rat had several chambers with cells treated with TGF-β1-CBD and other control ones, with cells untreated during the culture period or with commercial TGF-β1. The wounds were sutured and disinfected with Betadine. Four weeks later (implantation period), the rats were killed by overdose of the anesthetic and the chambers removed for histological and radiographic study. The latter was performed with low-intensity X-rays. For the histological study, the chambers were fixed in Bounin or in formalin buffered at 10%. After fixation, the plastic ring of the chambers was dissolved by sinking in acetone. After dehydration, they were embedded in paraffin and cut sagittally at several levels, at 5 micrometers of thickness. The sections were stained by different histochemical or immunocytochemical techniques with antibodies against different collagen types in order to analyze the tissue formed inside the chambers during the implantation period.

Figure 2 shows radiographic and histological images of different chambers demonstrating the cartilaginous and bone nature of the tissue formed in them. As compared to the control chambers where fibrillar tissue is hardly seen, the experimental ones show large tissue surfaces with cartilaginous characteristics, together with others where the osteoid matrix appears to be the prelude of a fully formed bone tissue. The data suggest that the cartilaginous tissue appears to be transformed into osteoid-bone, so this appears to indicate that an actual endochondral ossification process occurs inside the chambers.

### Example 7.- Example of implantation in a tibial fracture

Fragments of hydroxyapatite Proosteon 500 (Interopore) of about 0.5 cm of diameter were embedded with the cell suspension, dropping over them several microliters and changing their position to assure that most of their holes were filled with cells. The cells thus arranged were moved to the operating room, with care for asepsis and a temperature slightly over 0°C. After anesthetizing the patient, the fracture surfaces were prepared and the hydroxyapatite fragments were introduced separately. This fracture was actually a non-joining or atrophic pseudo-arthrosis, characterized by an avascular focus with fibrous tissue, with no proliferation of vascularized chondro-osteogenic tissue. Therefore, prior to the implant, and at the same surgical act, the fibrous tissue was removed from the fracture edges, leaving both surfaces in sclerotic bone tissue and perforating them until finding respectively proximal and distally a vascular source. The fragments of hydroxyapatite were placed in the holes, peripheral to the fracture focus on lay. The other hydroxyapatite fragments up to a total of 95 were placed in the fracture line. After placing the fragments of hydroxyapatite with the chondro-osteocompetent cells, both bone fragments were stabilized on a rigid, non-dynamic basis by a titanium plate attached by compression with titanium screws, complying with a neutralization principle.

Figure 3 shows images of histological sections performed in biopsy after six weeks of implantation. They show the neoformed tissue in contact with the hydroxyapatite walls with bone tissue features, and the formation of marrow tissue in the hollows left between the mineral trabeculae. The evidence provided shows that the tissue formed has characteristics of maturing bone. Furthermore, the images show that osteoinduction reaches the near implants, but not much farther, which can be explained because the cells transplanted are responsible for this osteoinduction, and extend where the cells spontaneously diffuse in the implant area. The area farther from the implant does not show symptoms of osteoinduction.

## Claims

1. Use of human mesenchymal stem cells differentiated to the chondro-osteogenic lineage by amplification with human serum and a transforming growth factor-beta 1 with a molecular domain for binding to collagen I (TGF-β1-CBD) obtained in eukaryotic expression systems in the preparation of a medicine for bone and/or cartilage repair.

2. The use according to claim 1, **characterized by** the administration of this medicine by injection in situ, in the systemic circulation or by implants, adsorbed in the appropriate biomaterial.

3. The use according to any of claims 1 to 2, **characterized in that** the administration is performed by implants, adsorbed in the appropriate material.

4. The use according to any of claims 1 to 3, **characterized in that** the material used for the implant is hydroxyapatite or diffusion chambers.

5. The use according to any of claims 1 to 4, **characterized in that** human mesenchymal stem cells are induced using a glucocorticoid.

6. The use according toclaim 5, **characterized in that** the glucocorticoid is dexamethasone.

7. The use according to any of claims 5 to 6, **characterized in that** β-glycerophosphate is additionally used the induction.

8. The use according to any of claims 1 to 7, **characterized in that** the transforming growth factor-beta 1 with a molecular domain for binding to collagen I has been expressed in eukaryotic expression systems.

9. The use according to claim 8, **characterized in that** the expression system used are insect cells.

10. The use according to claim 9, **characterized in that** the insect cells are transfected with baculovirus.

11. A procedure of capacitation of chondro-osteogenic cells in vitro from human mesenchymal stem cells, **characterized in that** it comprises the following steps:
a. the primary culture of stem cells in a medium supplemented with human serum and a transforming growth factor-beta 1 with a molecular domain for binding to collagen I (TGF-β1-CBD);
b. subsequent amplification by supplementation with human serum and TGF-β1-CBD;
c. the chondro-osteogenic induction with dexamethasone and β-glycerophosphate.

12. The procedure according claim 11, **characterized in that** the transforming growth factor-beta 1 fused to a molecular domain for binding to collagen I being expressed in eukaryotic expression systems.

13. The procedure according to claim 12, **characterized in that** the expression system used are insect cells.

14. The procedure according to above claim 13, **characterized in that** the insect cells are transfected with baculovirus.

15. The procedure according to any of claims 11 to 14 **characterized in that** the culture medium is a human type I collagen gel.

16. The procedure according to any of claims 11 to 15, **characterized in that** the initial supplementation of culture medium is performed with an amount between 0.1% and 1% of human serum, preferably 0.5%.

17. The procedure according to any of claims 11 to 16, **characterized in that** the amplification occurrs during a period ranging between 1 and 25 days, during which the culture medium is supplemented with an amount between 5% and 25% of human serum, preferably 20%.

18. Cells with chondro-osteogenic capacity obtainable according to the capacitation procedure of any of the claims 11 to 17.

19. Use of the cells with chondro-osteogenic capacity according to claim 18, in the preparation of a composition for bone and/or cartilage repair in humans.

20. Composition capable of inducing osteogenesis **characterized by** comprising a biocompatible, osteoconductive material, and a group of cells with chondro-osteogenic capacity obtained according to the capacitation procedure of any of the above claims 11 to 17.
